(19) Europäisches Patentamt
European Patent Office
Office européen des brevets

(11) EP 1 520 486 A1

(12) EUROPEAN PATENT APPLICATION

(43) Date of publication:
**06.04.2005 Bulletin 2005/14**

(51) Int Cl.[7]: **A23L 1/308**, A61P 3/04

(21) Application number: **03022007.3**

(22) Date of filing: **30.09.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **Tiense Suikerraffinaderij N.V.**
**1150 Brussel (BE)**

(72) Inventors:
- **Delzenne, Nathalie Maria**
**1970 Wezembeek-Oppem (BE)**
- **Cani, Patrice Daniel**
**1932 Zaventem (BE)**
- **Frippiat, Anne**
**1933 Sterrebeek (BE)**

(74) Representative: **Hermans, Johny**
**Tiense Suikerraffinaderij N.V.,**
**Aandorenstraat 1**
**3300 Tienen (BE)**

Remarks:
Claims 14-20. are deemed to be abandoned due to non-payment of the claims fees (Rule 31 (2) EPC).

# (54) Composition for suppressing ghrelin and method for same

(57) The use is provided of a fermentable dietary fibre for the manufacture of a composition comprising a combination of said fermentable dietary fibre and a food product for suppressing plasma ghrelin levels in humans and mammals, for the inhibition of the synthesis and/or release and the effects of the appetite-stimulating hormone ghrelin in humans and mammals. The composition is also suitable for the prevention and inhibition of the development of obesity and obesity-related diseases, as well as for the treatment of obesity, in humans and mammals.

Furthermore, a method is provided for suppressing plasma ghrelin levels, for inhibiting the synthesis and/or release and the effects of the appetite-stimulating hormone ghrelin, in humans and mammals. Also a method is provided for the prevention and inhibition of the development of obesity and obesity-related diseases, as well as for the treatment of obesity, in humans and mammals. The method involves oral intake or administration through tube feeding of a composition comprising a combination of a fermentable dietary fibre and a food product.

**Fig. 5**

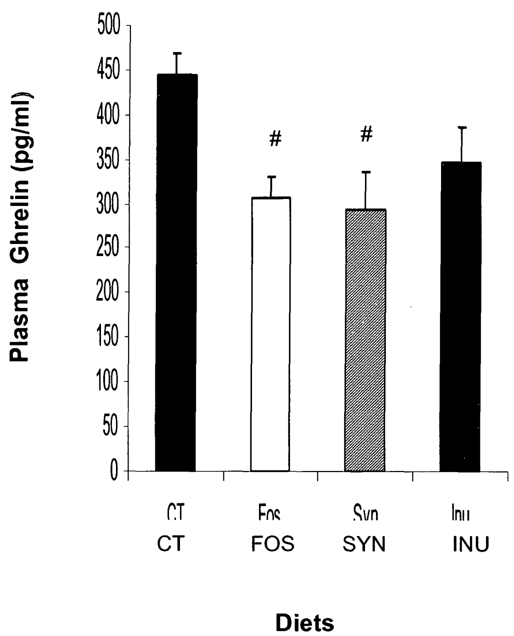


Mean value +/- ESM, n = 6. (#) significant *versus* CT ($p$<0.05)
Diets: CT : Control group (Feed UAR-AO4)
FOS : UAR-AO4 + 10 % FOS (RAFTILOSE® P95)
SYN : UAR-AO4 + 10 % RAFTILOSE® SYNERGY1
INU : UAR-AO4 + 10 % Inulin (RAFTILINE® HP)



Printed by Jouve, 75001 PARIS (FR)

## Description

### Field of the invention

The present invention relates to compositions comprising fermentable dietary fibres for the suppression of plasma ghrelin levels in humans and mammals, and the resulting inhibition of food intake, obesity and obesity-related diseases, as well as to a method for same.

### Background and prior art

As a result of the increased availability of high caloric food products, obesity has sharply increased over the last few decennia in developed as well as in developing countries. According to the World Health Organisation (WHO) more than 20 % of the people in developed countries and about 5 % of the people in developing countries are actually overweight.

The notions overweight and obesity relate to the ratio of height and weight of the subject expressed as Body Mass Index (BMI), following the formula BMI = W/HxH wherein W represents the weight in kg and H represents the height in meter. For humans, a BMI between 20 and 25 is considered normal, a BMI between 25 and 30 is considered as overweight and a BMI 30 or more is considered as obesity.

Since the conditions of overweight gradually move to conditions of obesity, and concurrently, the undesirable effects on health caused by overweight gradually move to the undesirable effects on health caused by obesity, no differentiation is made hereinafter between overweight and obesity. Accordingly, both conditions, overweight and obesity, are referred to hereinafter by the term obesity.

Apart from esthetical aspects, obesity has been found to have undesirable effects on health. The sharp increase of obesity over the last decades has been recognised as a major cause of the concurrently sharp increase of various undesirable health conditions and diseases in humans. Obesity has indeed been identified as an important factor in the development and evolution of various diseases, including cardiovascular diseases, hyperglycemia, diabetes, steatosis, hypertriglyceridemia, osteoarthritis, hypertension, lung disease and cancer, particularly bowel and breast cancer.

Because obesity is still steadily increasing, it tends to become a pandemic that threatens health and well-being of a large part of the population worldwide.

Furthermore, analogue to humans, domesticated mammals, particularly pets such as dogs and cats, appear to present a similar pattern with respect to the development and evolution of obesity and obesity-related undesirable conditions and diseases.

In view of the health threats resulting from obesity, significant research efforts are continuously made worldwide to elucidate the mechanisms leading to obesity and to find ways for preventing, inhibiting and treating obesity and obesity-related diseases.

Research results revealed that food intake in humans and mammals is regulated by the central nervous system, particularly in the hypothalamus, and that several hormones are involved in feeding behaviour (including the regulation of food intake, of appetite and of satiety feelings), in food metabolism, in biosynthesis and storage of fat in various organs, as well as in the development, evolution and maintaining of obesity.

Several appetite-regulating hormones have already been identified, revealing that various complex mechanisms are involved in the regulation of food intake. Furthermore, complex interactions have already been discovered between hormones that control food intake, metabolism, fat synthesis, fat storage, appetite, feeding behaviour and satiety feelings in humans and mammals.

Feeding behaviour appears largely regulated by a balance between food intake-stimulating hormones (*i.e.* appetite-stimulating hormones) and food intake-inhibiting hormones (*i.e.* appetite-inhibiting hormones).

Identified food intake-stimulating hormones comprise *i.a.* neuropeptide Y (NPY), agouti-related peptide (AgRP), and the recently discovered ghrelin, and food intake-inhibiting hormones comprise *i.a.* leptin and glucagon-like peptide-1 (GLP-1).

Ghrelin is described by K. Kojima et al., (Current Opinion in Pharmacology, Ed. Elsevier Science Ltd., Vol.2, 665-668, 2002) as an orexigenic signalling molecule. Ghrelin is disclosed to be an appetite-regulating hormone with a peptide structure that is produced mainly in the stomach, and ghrelin peptides have already been identified in several mammals. Rat and human ghrelin peptides were found to consist both of 28 amino acids, differing only in two amino acid residues. Both ghrelin peptides are bearing a n-octanoyl group at the Ser 3 amino acid unit, a structure which is essential for the activity of ghrelin.

Ghrelin was found to be appetite-stimulating when administered centrally as well as peripherally.

The main active site of ghrelin was found to be the hypothalamic arcuate nucleus, which is also the target site of leptin (an appetite-suppressing hormone from adipose tissues), as well as of neuropeptide Y (NPY) and agouti-related protein (AgRP) (both appetite-stimulating peptides produced in the arcuate nucleus). The appetite-stimulating effects of both NPY and AgRP were found to be inhibited directly by leptin. The appetite-stimulating effect of ghrelin was found to be blocked by an NPY receptor antagonist. Intravenous (IV) injection of ghrelin was found to stimulate neurons that contained NPY and/or AgRP in the hypothalamus (M. Kojima et al., *o.c.*, p. 666-667) .

These results indicate that ghrelin increases feeding activity by stimulating NPY- and AgRP-containing neurons in the hypothalamus to promote the production and secretion of NPY- and AgRP peptides.

Accordingly, ghrelin was found to stimulate food intake and to be a natural antagonist to leptin (M. Kojima et al., *o.c.*, p. 667).

Regulation of ghrelin secretion appears to be controlled by feeding. In healthy humans, plasma ghrelin concentrations increase before meals and during periods of fasting, inducing hunger, and they decrease after food intake, reaching a nadir within about one hour after meals.

Furthermore, patients who have undergone gastric bypass lose approximately 36 % of their weight with a concurrent decrease of their plasma ghrelin concentration (M. Kojima et al., *o.c.*, p. 667).

J. Nedvidkova et al. (Journal of Clinical Endocrinology and Metabolism, Vol. 88(4), 1678-1682, 2003) describe investigations regarding the responses of plasma ghrelin to food intake, meal volume and meal nutritional value in healthy woman. After overnight fasting all subjects received either a standardised breakfast (containing fat, proteins and carbohydrates) or the non-caloric fibre Psyllium. In healthy women, following the standardised breakfast, plasma ghrelin concentrations were decreasing till about two hours after the meal, whereas after the intake of Psyllium, plasma ghrelin concentrations were already markedly decreased 30 minutes after consumption and remained low, at about the level reached after 90 minutes by the standardised breakfast intake, for the next 90 minutes.

J. Nedvidkova et al. concluded that consumption of a standardised (2439 kJ [585 kcal]) breakfast or of Psyllium fibre was equally effective in suppressing plasma ghrelin in healthy women. According to Nedvidkova et al., these results suggest that the acute response of plasma ghrelin to feeding is independent of meal nutritional value, but is rather related to the presence of food in the stomach. Furthermore, because plasma ghrelin was found to decrease also after intravenous administration of glucose, it was concluded that ghrelin secretion is not influenced by stomach expansion (J. Nedvidkova et al., *o.c.*, p. 1680).

The importance of ghrelin in the control of feeding behaviour in humans is also evidenced by the occurrence of altered levels of ghrelin in healthy subjects following fasting and food intake. Plasma ghrelin concentrations of normal subjects show a diurnal pattern with preprandrial increases and postprandrial decreases (T. Shiiya et al., Journal of Clinical Endocrinology and Metabolism, Vol. 87(1), 240-244, 2002).

From the prior art, it can thus be concluded that ghrelin is a gastro-intestinal released hormone that circulates in the bloodstream and transmits appetite-stimulating signals from peripheral, gastro-intestinal organs to the brain, in particular to the appetite-regulating regions in the hypothalamus. Furthermore, it can be concluded that energy and metabolic processes in humans and mammals may be controlled by modulating a balance between factors or hormones that regulate appetite in a negative and a positive manner.

In view of the steadily increase of obesity in humans and in domesticated mammals, particularly pets, and the undesirable effects provoked on health by obesity, there is clearly a need to prevent and inhibit the development of obesity and to treat conditions of obesity, in order to maximally prevent or reduce the development and effects of obesity-related undesirable conditions and diseases in humans and domesticated mammals.

It is the aim of the present invention to provide a solution for one or more of the above-mentioned problems.

<u>Object and description of the invention</u>

Whereas J. Nedvidkova et al. (o.c., p. 1680, left column) disclose that, after overnight fasting, the consumption of a standardised breakfast (containing fat, proteins and carbohydrates) or non-caloric Psyllium fibre were about equally effective in reducing plasma ghrelin levels in healthy subjects during a certain period of time after food-intake, named post-absorptive or postprandial period, the inventors have surprisingly found that, as the result of the intake of a composition comprising a combination of a food product and a fermentable dietary fibre, the plasma ghrelin level in rats remains lower in the post-absorptive period for at least 8 hours, compared to controls. Controls herein is meant subjects that have consumed only said food product and did not consume any fibres except the ones (non-fermentable cellulose) present in said food product that constituted the normal diet. Said findings have led the inventors to the present invention.

In view of the analogy between rats and humans with respect to ghrelin, said findings provide the possibility to suppress plasma ghrelin levels in humans and mammals, comprising to inhibit the synthesis and/or release of ghrelin, and consequently to suppress the effects of the appetite-stimulating ghrelin hormone, to control food-intake behaviour, particularly by the suppression of food-intake, and to inhibit the development of obesity and obesity-related conditions and diseases in humans and mammals. The terms to inhibit and to suppress, and the corresponding terms suppression and inhibition are interchangeably used herein.

Accordingly, the present invention provides in one aspect the use of a fermentable dietary fibre for the manufacture of a composition for oral administration or tube feeding, comprising a combination of said fermentable dietary fibre and a food product, for suppressing plasma ghrelin levels in humans and mammals.

In another aspect, the present invention provides the use of a fermentable dietary fibre for the manufacture of a composition for oral administration or tube feeding comprising a combination of said fermentable dietary fibre and a food product, for maintaining a lower plasma ghrelin level in humans and mammals, compared to controls having consumed only said food product, in the post-absorptive period for at least 8 hours.

In another aspect, the present invention provides the use of a fermentable dietary fibre for the manufacture of a composition for oral administration or tube feeding comprising a combination of said fermentable dietary fibre and a food product, for the inhibition of the synthesis or release of ghrelin, as well as for the inhibition of the effects of the appetite-stimulating hormone ghrelin in humans and mammals.

In a further aspect, the present invention provides the use of a fermentable dietary fibre for the manufacture of a composition for oral administration or tube feeding comprising a combination of said fermentable dietary fibre and a food product, for the control of food-intake behaviour, in particular for the inhibition of food-intake.

In still another aspect, the present invention provides the use of a fermentable dietary fibre for the manufacture of a composition for oral administration or tube feeding comprising a combination of said fermentable dietary fibre and a food product, for the prevention, reduction of the risk of development, or inhibition of the development of obesity and obesity-related diseases in humans and mammals.

In still a further aspect, the present invention provides the use of a fermentable dietary fibre for the manufacture of a composition for oral administration or tube feeding comprising a combination of said dietary fibre and a food product, for the treatment of obesity in humans and mammals.

In another aspect, the present invention provides a method for suppressing plasma ghrelin levels in humans and mammals by administration orally or through tube feeding to a being in need of such treatment of an effective amount of a composition comprising a combination of a fermentable dietary fibre and a food product.

In another aspect, the present invention provides a method for maintaining a lower plasma ghrelin level in humans and mammals, compared to controls having consumed only said food product, in the post-absorptive period for at least 8 hours, by administration orally or through tube feeding to a being in need of such treatment of an effective amount of a composition comprising a combination of a fermentable dietary fibre and a food product.

In a further aspect, the present invention provides a method for the inhibition of the synthesis or release, as well as for the inhibition of the effects of the appetite-stimulating hormone ghrelin in humans and mammals by administration orally or through tube feeding to a being in need of such treatment an effective amount of a composition comprising a combination of a fermentable dietary fibre and a food product.

In still a further aspect, the present invention provides a method for the control of food-intake behaviour, in particular for the inhibition of food-intake, in humans and mammals by administration orally or through tube feeding to a being in need of such treatment of an effective amount of a composition comprising a combination of a fermentable dietary fibre and a food product.

In still a further aspect, the present invention provides a method for the prevention, reduction of the risk of development, or inhibition of the development of obesity and obesity-related diseases in humans and mammals by administration orally or through tube feeding to a being in need of such treatment of an effective amount of a composition comprising a combination of a fermentable dietary fibre and a food product.

In an ultimate aspect, the present invention provides a method for the treatment of obesity in humans and mammals by administration orally or through tube feeding to a being in need of such treatment of an effective amount of a composition comprising a combination of a fermentable dietary fibre and a food product.

Mammals refers herein to domesticated mammals, comprising in particular pets, such as dogs and cats.

According to one embodiment, the composition of the invention consists of said combination of a fermentable dietary fibre and a food product. According to another embodiment, the composition of the invention comprising said combination of a fermentable dietary fibre and a food product, contains one or more further components, for example, a probiotic, a food supplement, vitamins, minerals, trace elements, and/or a drug.

Fermentable dietary fibres are well known in the art. This term refers to carbohydrates that are resistant to hydrolysis by the enzymes of the digestive tract of humans and mammals, but that are fermented by bacteria in the large intestine, particularly bacteria of the genus Bifidus and the genus Lactobacillus. Fermentation is the process whereby the bacteria utilise the carbohydrate as their primary energy source and metabolise it, thereby producing gases and short-chain fatty acids as by- products.

The term fermentable dietary fibre herein embraces soluble fibres as well as insoluble fibres (solubility in water at 25 °C, respectively, equal or more than 1 g/l and less than 1 g/l) that can be oligosaccharides as well as polysaccharides. The difference between an oligosaccharide and a polysaccharide is based on the number of saccharide units building up the saccharide molecule. Conventionally, and also herein, a saccharide containing less than 10 saccharide units is named an oligosaccharide, whereas the term polysaccharide refers to a carbohydrate that is built of 10 or more saccharide units. Oligosaccharides can be products of nature as well as products derived from products of nature, for example by partial, enzymatic or acidic hydrolysis. Oligosaccharides can also be obtained by enzymatic synthesis from saccharides, by enzymatic transformation of saccharides, and by chemical synthesis or chemical transformation of saccharides.

It is understood that the term fermentable dietary fibre herein includes a mixture of fibre molecules of the same degree of polymerisation (homodisperse mixture), a mixture of dietary fibre molecules of different degree of polymerisation (polydisperse mixture), as well as a mixture of two or more dietary fibres of different chemical structure. The term different chemical structure herein includes linear and branched molecules composed of the same saccharide units, as well as molecules composed of different saccharide units.

In a preferred embodiment, the term fermentable dietary fibre refers herein to a carbohydrate selected from the group consisting of pectins, including for example arabinans, galacturonans, galactans and arabinogalactans; hemi-celluloses, comprising for example xylanes and beta-glucans; gums, comprising for example arabic gum, locust bean gum, guar gum and tragacanth; galactomannans; carrageenans; agar; alginates; fructans, comprising levan, inulin and oligofructose; and waxes; derivatives of said carbohydrates, for example resistant starch, oligo-fructosaccharides, oligo-galactosaccharides, and oligo-xylosaccharides; as well as any mixture of said fibres.

In a more preferred embodiment, the dietary fibre is inulin or oligofructose (also termed interchangeably oligo-fructosaccharide or fructo-oligosaccharide), which are both well known in the art.

Inulin is commonly obtained by extraction followed by purification according to conventional methods from plant sources, mainly from roots of chicory (*Cichorium intybus*), tubes of Jerusalem artichoke (*Helianthus tuberosus*) and Dahlia, and from the piña (head) of the Blue Agave plant.

Oligofructose (fructo-oligosaccharide) is commonly obtained by partial hydrolysis of inulin, as well as by enzymatic synthesis from saccharose.

In an even more preferred embodiment, the dietary fibre is chicory inulin or oligofructose obtained from chicory inulin, preferably by partial enzymatic hydrolysis, or any mixture thereof.

Chicory inulin has a degree of polymerisation, namely the number of saccharide units in an inulin molecule, ranging from 2 to about 70. Oligofructose has typically a degree of polymerisation ranging from 2 to 9. Inulin as well as oligofructose is composed of polyfructose molecules that essentially consist of fructose units that are linked to each other mostly or exclusively by beta(2-1) fructosyl-fructose linkages. The polyfructose chain may or may not end in a glucose unit. Thus inulin and oligofructose correspond to the general formula GFn or Fm, wherein G represents a glucose unit, F represents a fructose unit, and m and n are integers from 2 onwards. A characterising parameter of inulin and oligofructose is the degree of polymerisation (represented by DP), corresponding to the value of n + 1 or m. Another characterising parameter is the (number-)average degree of polymerisation, represented by av. DP or ($\overline{DP}$), which is the value corresponding to the total number of (G and F) saccharide units divided by the total number of inulin molecules present in a given sample, without taking into account the monomeric glucose and fructose and the dimeric saccharose that is present in the sample (H. Hoebregs, J. AOAC International, Vol.80(5), 1029-1037,1997).

Chicory inulin, obtained at industrial scale from roots of chicory, as well as oligofructose, typically obtained by partial enzymatic hydrolysis of chicory inulin, are available in various commercial grades, for example as RAFTILINE® (Trade name for chicory inulin) and RAFTILOSE® (Trade name for oligofructose) from ORAFTI, Tiense Suikerraffinaderij n.v., Belgium.

Commercial grades of inulin comprise RAFTILINE® ST, (with an av. DP of at least 10, typically of 10 to 13, and containing in total about 8 wt % glucose, fructose and sucrose), RAFTILINE® LS (with an av. DP of at least 10, typically of 10 to 13, but containing in total less than 1 wt% glucose, fructose and sucrose), RAFTILINE® HP (which has an av. DP of at least 23, typically of 23 to 25, and is essentially free of glucose, fructose and sucrose).

Commercial grades of oligofructose comprise RAFTILOSE® P95 which contains about 95 % by weight oligofructose with a DP ranging from 2 to 9 and which contains about 5 % by weight in total of glucose, fructose and sucrose.

Further suitable grades of inulin include particular mixtures of oligofructose and long-chain inulin (inulin with av. DP of at least 20, typically of 23 to 25), in a weight ratio ranging from 10/90 to 70/30, with a total content of inulin with a DP 9 and DP 10 of maximally 5 %, preferably maximally 3 % or even maximally 2 %, by weight, described in patent application WO 01/60176. Such inulin grade is commercially available from ORAFTI (Belgium), for example as RAFTILOSE® Synergyl wherein said weight ratio of oligofructose and long-chain inulin of av. DP of 23 to 25 is about 1/1.

Agave inulin is commercially available for example as GAVEDIET®PR with av. DP of 14-16 from Industrias Colibri Azul S.A. de C.V., Mexico.

Fructo-oligosaccharide (FOS) obtained by enzymatic synthesis from saccharose is for example commercially available as ACTILIGHT® 950P (with ≥ 95% of DP 3-5) from Béghin-Meiji Industries.

The term food product herein refers to any food product, typically to a conventional food product, to a conventional feed product, as well as to a mixture of two or more components of such conventional food or feed products. This mixture of two or more components may constitute an intermediate that is suitable for the manufacture of a food or feed product. Typically, the food product contains proteins, carbohydrates and fat components in a conventional balance. The food product may be present in any conventional form, such as a liquid, a paste, a shaped solid. When said combination is composed of a mixture of a fermentable dietary fibre and two or more components of such conventional food or feed products, it may be present too in any form, such as a liquid, a paste, a powder, or a shaped solid.

The combination of the fermentable dietary fibre and the food product, and the composition according to the invention, can be obtained by conventional manufacturing processes, such as, for example, mixing of the fermentable dietary fibre and the food or feed product, dry or wet mixing of the components, extrusion of a mixture of the components, and co-spray-drying of the components of the combination or of the composition.

In case the composition comprises, apart from the combination of the fermentable dietary fibre and the food product, an additional component, such composition can also be prepared by conventional techniques, for example by dry or wet mixing of all components at once, or by including the additional component into the separately prepared combination of the fermentable dietary fibre and the food product, or by mixing the additional component, the fermentable fibre and the food or feed product according to conventional techniques.

The term food product herein embraces both food product (intended for human consumption) and feed product (intended for animal consumption).

The composition according to the invention contains the combination of fermentable dietary fibre and food product in such a concentration that the intake of the composition provides an effective amount of said combination to the subject.

By effective amount is meant an amount that provokes a statistically significant suppression of plasma ghrelin levels in humans and mammals compared to controls having consumed only said food product, and that, accordingly, provokes the maintaining of a statistically significant lower plasma ghrelin level in humans and mammals compared to controls in the post-absorptive period, preferably for at least 8 hours. The effective amount provokes a plasma ghrelin concentration in the subject in the post-absorptive period after 8 hours that is preferably at least 30 % lower, more preferably at least 50 % lower, than the plasma ghrelin concentration of the controls.

In the said combination, the weight ratio fermentable dietary fibre / food product may range from 1/99 to 50/50, preferably from 2/98 to 30/70.

The preferred daily intake by humans of the composition provides a daily intake of fermentable dietary fibres ranging from 3 g to 50 g, preferably from 3 g to 30 g, more preferably from 5 g to 15 g.

The daily intake of the composition can be obtained by one single dose intake or by the intake of partial units spread over a one-day period that together corresponds to the desired total daily intake.

In a particular embodiment, the composition according to the present invention may be present in the form of a two-pack. Accordingly, the dietary fibre component may be present as such in a form, or in combination with an additional component, for example a drug, in a galenic form, separately from the food product. Both components of the two-pack form are then taken or administered in such a manner that they are simultaneously present in the body so that they interact and provide the decreasing effect on plasma ghrelin levels that corresponds to the effect of the corresponding composition when present in a one-pack form.

The invention is illustrated by the experiments given below and results obtained in the experiments are shown in Figures 1 to 5, wherein the abbreviations used are:

CT : Control group receiving standardised feed (UAR-A04)

FOS: group receiving a composition consisting of standard

feed (UAR-A04) + 10wt% oligofructose (RAFTILOSE® P95)

SYN: group receiving a composition consisting of standard

feed (UAR-A04) + 10wt% mixture of oligofructose + inulin (RAFTILOSE®SYNERGY1)

INU: group receiving a composition consisting of standard

feed (UAR-A04) + 10wt% inulin(RAFTILINE®HP)

<u>Figure 1</u> shows the plasma ghrelin concentration in rats fed a standardised feed and the standardised feed + 10 wt % FOS in the post-absorptive period 8 hours after the last meal.

<u>Figure 2</u> shows the weight of rats fed a standardised feed without and with fermentable dietary fibres in the post-absorptive period 8 hours after the last meal.

<u>Figure 3</u> shows the evolution of the energy consumption of rats fed a standardised feed without and with fermentable dietary fibres during the experiment till sacrifice. Measurements were made at regular intervals, twice a week.

<u>Figure 4</u> shows the weight of the epidydimal fat tissue, in % of the body weight, of rats fed standardised feed without and with fermentable dietary fibres at the sacrifice of the animals 8 hours after the last meal.

<u>Figure 5</u> shows the plasma ghrelin concentration in rats fed standardised feed without and with fermentable dietary fibres at the sacrifice of the animals 8 hours after the last meal.

<u>Experiment 1</u>

In a first experiment, normoglycemic Wistar male rats (groups of 12 rats) received either a standardised diet (feed UAR-A04 in powder form) (control [CT] group) or a standardised diet to which 10 % by weight oligofructose (FOS) (RAFTILOSE® P95) was added. After 3 weeks of treatment with either the standardised diet (CT group) or the same diet enriched with 10 % by weight oligofructose (FOS group) at a mean daily energy intake/rat throughout the treatment of 75 to 80 kcal/day for the CT group and 65 to 68 kcal/day for the FOS group, the rats were fasted for 8 hours before blood sample collection under anaesthesia.

Plasma ghrelin concentration was measured according to the method described by Linco Research, Active Ghrelin RIA Kit, USA.

The results are presented in Figure 1 below, and show that the plasma ghrelin concentration was significantly lower 8 hours after the last meal in rats fed meals containing fermentable dietary fibres (FOS group), than in the control rats (CT group).

Accordingly, the lower plasma ghrelin concentration in the group fed meals containing fermentable dietary fibres can be considered as an indicator of satiety maintenance, effecting food-intake behaviour by postponing the impulses and tendency for food intake compared to the control group.

Experiment 2

In a second experiment, carried out on normoglycemic Wistar male rats, the effects of various fermentable dietary fibres, including oligofructose [RAFTILOSE® P95] (FOS group), inulin [RAFTILINE® HP] (INU group), and a mixture of oligofructose-inulin [RAFTILOSE® Synergyl] (SYN group), added at 10 % weight to a standardised food, were compared to the standardised food [UAR-AO4 in powder form] (CT group).

The methodology corresponds to the one indicated in Example 1.

The results are presented in Figure 5 below and show that, compared to a diet of standard food, diets of a composition containing a combination of the standard food product and respectively the fermentable dietary fibres RAFTILINE® HP (inulin), RAFTILOSE® P95 (oligofructose) and particularly RAFTILOSE® Synergyl (a particular mixture of oligofructose-inulin), are able to suppress and maintain lower levels of plasma ghrelin concentrations.

Furthermore, the results, presented below in Figures 2 to 5, show clearly that, compared to rats from the control group, rats of the groups fed a composition containing a combination of the standard feed and said dietary fibres, present:

- a lower weight gain at the end of the experiment (Figure 2),
- a lower energy consumption over the duration of the experiment (Figure 3),
- a lower weight of the epidydimal fat tissue at the end of the experiment (Figure 4),
- a reduced plasma ghrelin level 8 hours after the last food intake. Ghrelin levels were more reduced in the group fed a composition comprising oligofructose than in the group fed a composition comprising inulin, and were most reduced in the group fed a composition comprising RAFTILOSE®Synergyl (Figure 5).

Furthermore, the food-intake by rats is known to be driven by calories-intake provided by the food taken, not by the weight of the food taken. Moreover, rat intestinal flora is assumed to be adapted to the diets in about a week after the start of the experiments. Accordingly, from Figure 3 it follows that intake of the compositions of the invention provokes a significant reduction of the energy intake. So, it can be concluded that the compositions of the invention suppress food intake and hence have an inhibitory effect on the development of obesity and accordingly on obesity-related undesirable conditions and diseases. Furthermore, it can be concluded that the compositions of the invention are suitable for the treatment of obesity.

Conclusion.

The above results demonstrate that the consumption of compositions according to the present invention, comprising a combination of a fermentable dietary fibre and a food product as defined hereinabove, is suitable for suppressing plasma ghrelin levels and for maintaining post-absorptive reduced plasma ghrelin levels in humans and mammals compared to subjects that took only said food product without fermentable dietary fibres. The results also show that the compositions are able to inhibit the production and/or release of ghrelin in humans and mammals, and thus to provoke inhibiting effects on appetite and hence on food intake and energy intake. Hence, the compositions of the invention are suitable to prevent the development of obesity and thus also of obesity-related undesirable conditions and diseases, as well as to provoke a reduction of obesity and accordingly of obesity-related undesirable conditions and diseases.

These results also support the method of the present invention defined hereinabove for suppressing the plasma ghrelin concentration in humans and mammals, for inhibiting the synthesis and/or release of ghrelin, and for suppressing the effects of the appetite-stimulating hormone ghrelin, and, accordingly also the method for the prevention, inhibition and treatment of obesity and obesity-related undesirable conditions and diseases in humans and mammals, said method involving administration of a composition comprising a combination of a fermentable dietary fibre and a food product as defined hereinabove.

## Claims

1. Use of a fermentable dietary fibre for the manufacture of a composition for oral administration or tube feeding, comprising a combination of said dietary fibre and a food product, for suppressing plasma ghrelin levels in humans and mammals.

2. Use according to claim 1 of a fermentable dietary fibre for the manufacture of a composition for the inhibition of the synthesis, the release or the effects of the appetite-stimulating hormone ghrelin in humans and mammals.

**3.** Use according to claim 1 of a fermentable dietary fibre for the manufacture of a composition for the prevention or reduction of the risk of development of obesity and obesity-related diseases in humans and mammals.

**4.** Use according to claim 1 of a fermentable dietary fibre for the manufacture of a composition for the inhibition of the development of obesity and obesity-related diseases in humans and mammals.

**5.** Use according to claim 1 of a fermentable dietary fibre for the manufacture of a composition for the treatment of obesity in humans and mammals.

**6.** Use according to any one of claims 1 to 5, wherein the fermentable dietary fibre is a carbohydrate selected from the group consisting of pectins, comprising arabinans, galacturonans, galactans and arabino-galactans; hemi-celluloses, comprising xylanes and beta-glucans; gums, including arabic gum, locust bean gum, guar gum and tracanth; galactomannans; carrageenans; agar; alginates; fructans, and waxes, derivatives of said carbohydrates, including resistant starch, oligo-fructosaccharides, oligo-galactosaccharides, and oligo-xylosaccharides, as well as any mixture of said fibres.

**7.** Use according to claim 6, wherein the fermentable dietary fibre is inulin, oligofructose, fructo-oligosaccharide, or any mixture thereof.

**8.** Use according to claim 7, wherein the inulin is chicory inulin or oligofructose obtained from chicory inulin, or any mixture thereof.

**9.** Use according to any one of claims 1 to 8 of a fermentable dietary fibre for the manufacture of a composition wherein the weight ratio fermentable dietary fibre/food product ranges from 1/99 to 50/50.

**10.** Use according to claim 9, wherein the amount of composition for daily intake by humans contains an amount of fermentable dietary fibres ranging from 3 g to 50 g.

**11.** Use according to claim 10, wherein the amount of composition for daily intake by humans contains an amount of fermentable dietary fibres ranging from 5 g to 15 g.

**12.** Use according to any one of claims 1 to 11 wherein the composition is present in the form of a two-pack.

**13.** Use according to any one of claims 1 to 9, wherein the mammals are dogs or cats.

**14.** Method for suppressing plasma ghrelin levels in humans and mammals by administration orally or through tube feeding to a subject in need of such treatment of an effective amount of a composition comprising a combination of a fermentable dietary fibre and a food product defined in any one of claims 1 and 6 to 12.

**15.** Method according to claim 14, for the inhibition of the synthesis, the release or the effects of the appetite-stimulating hormone ghrelin in humans and mammals.

**16.** Method according to claim 14, for the prevention or reduction of the risk of development of obesity and obesity-related diseases in humans and mammals.

**17.** Method according to claim 14, for the inhibition of the development of obesity and obesity-related diseases in humans and mammals.

**18.** Method according to claim 14, for the treatment of obesity in humans and mammals.

**19.** Method according to any of claims 14 to 18 wherein the effective amount of composition for daily intake by humans provides an amount of fermentable dietary fibres ranging from 3 g to 50 g.

**20.** Method according to claim 19 wherein the effective amount of composition for daily intake by humans provides an amount of fermentable dietary fibres ranging from 5 g to 30 g.

Fig. 1

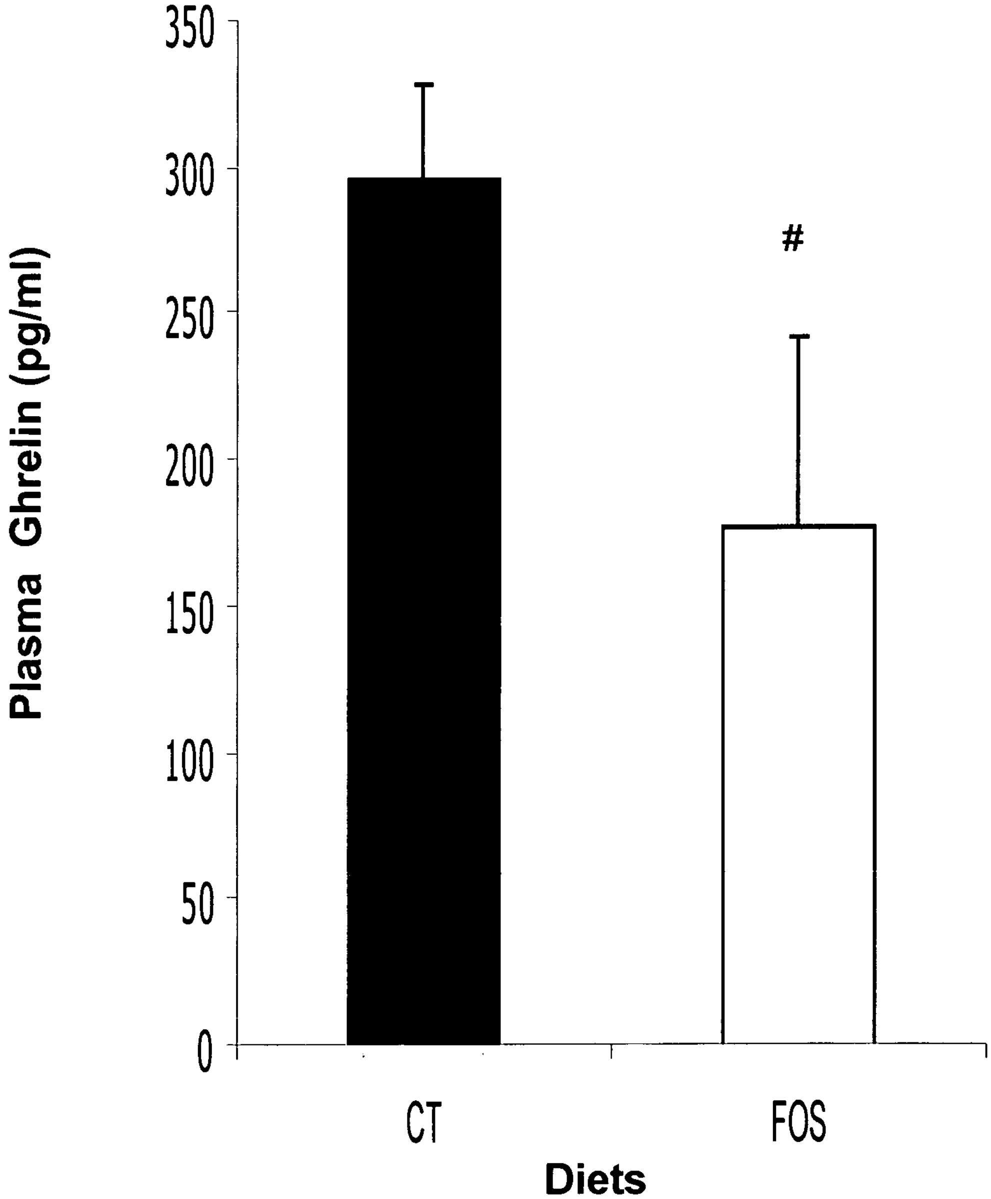


Diets: CT: Control group; FOS: FOS group (Feed + 10 % FOS )
Mean value +/- ESM, n = 6, (#) significant *versus* CT ($p$ = 0,02).

**Fig. 2**

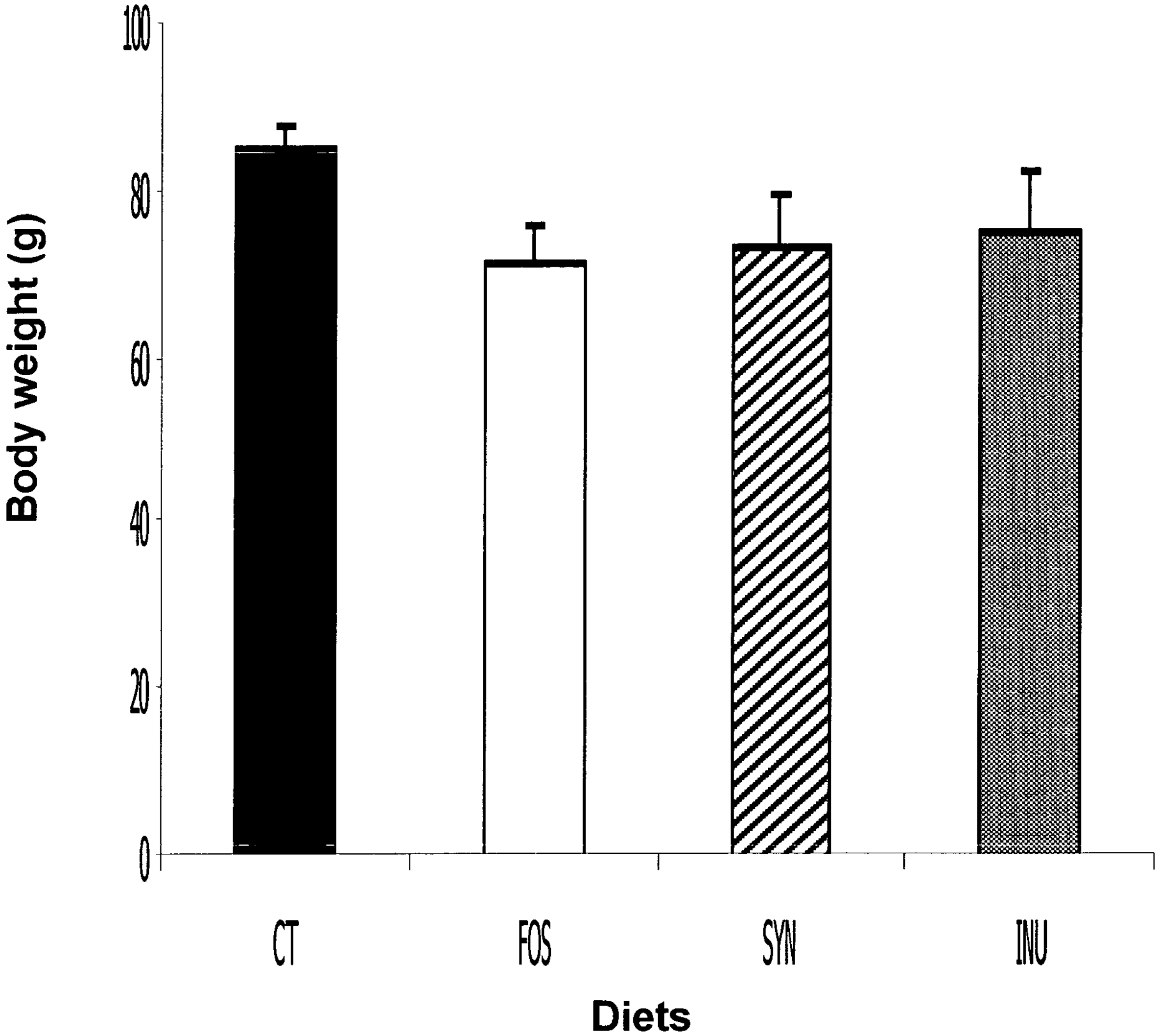


Mean value +/- ESM, n = 6.

Diets: CT : Control group (Feed UAR-AO4)

FOS : UAR-AO4 + 10 % FOS (RAFTILOSE® P95)

SYN : UAR-AO4 + 10 % RAFTILOSE® SYNERGY1

INU : UAR-AO4 + 10 % Inulin (RAFTILINE® HP)

**Fig. 3**

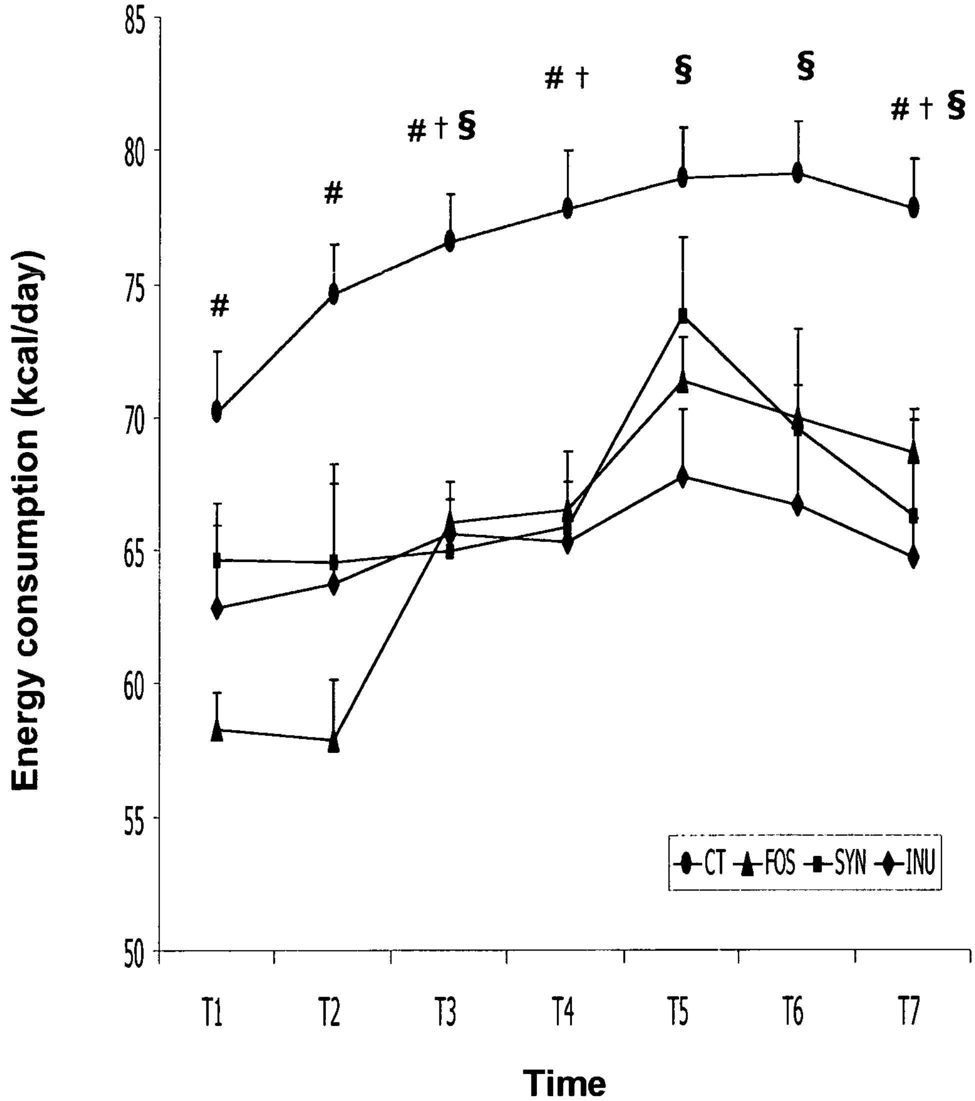


Mean value +/- ESM, n = 6.

Symbols : significant *vs* CT (*p* < 0,05) : (#) FOS, (†) SYN, (§) INU

Diets : CT : Control group (Feed UAR-AO4)

FOS : UAR-A04 + 10 % FOS (RAFTILOSE® P95)

SYN : UAR-A04 + 10 % RAFTILOSE® SYNERGY1)

INU : UAR-A04 + 10 % Inulin (RAFTILINE® HP)

Time : T1 to T6 : two measurements/week at regular intervals during week 1 to 3.

T7 : Time at sacrifice.

**Fig. 4**

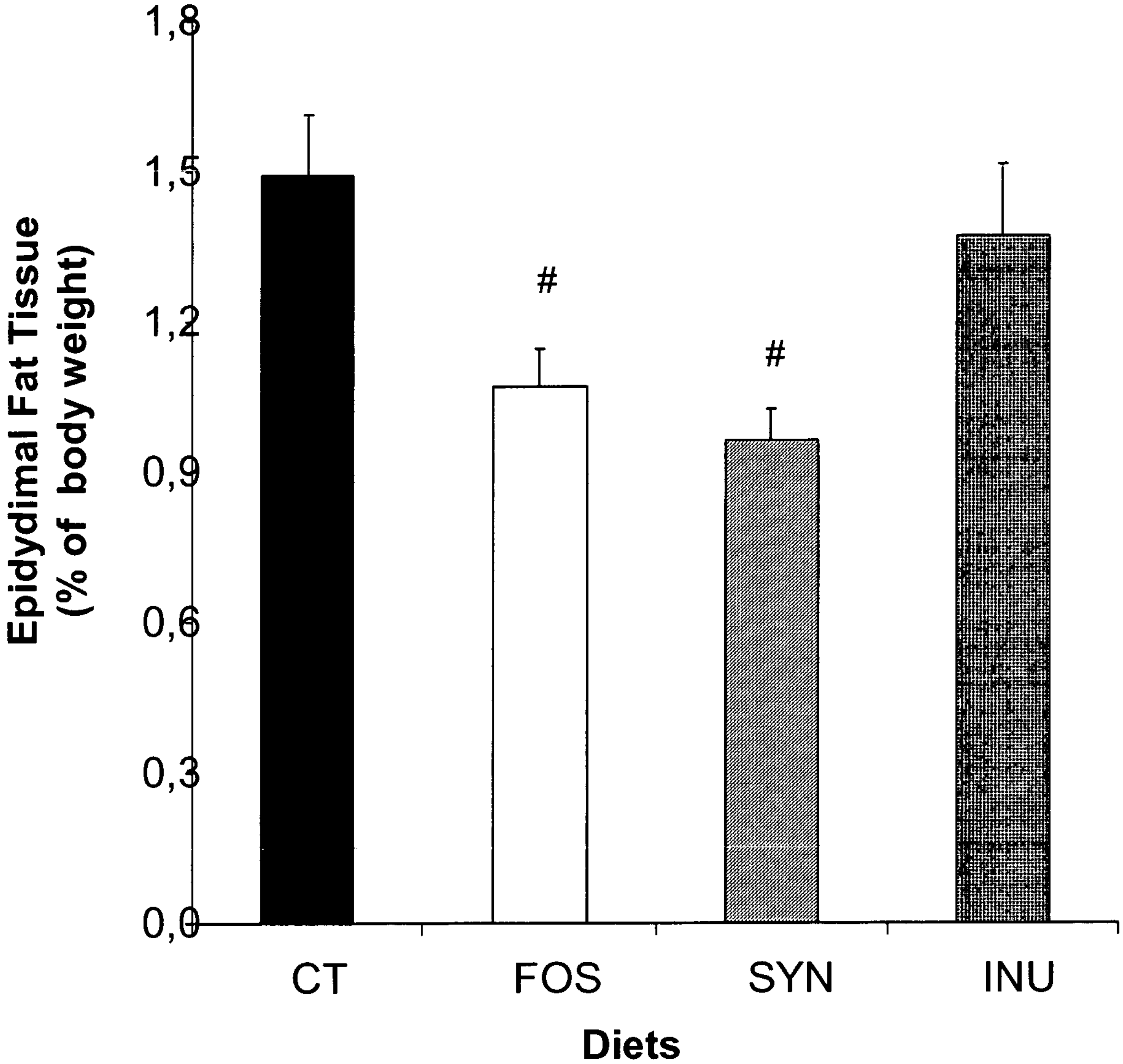


Mean value +/- ESM, n = 6. (#) significant *versus* CT ($p$<0.05)

Diets: CT : Control group (Feed UAR-AO4)

FOS : UAR-AO4 + 10 % FOS (RAFTILOSE® P95)

SYN : UAR-AO4 + 10 % RAFTILOSE® SYNERGY1

INU : UAR-AO4 + 10 % Inulin (RAFTILINE® HP)

Fig. 5

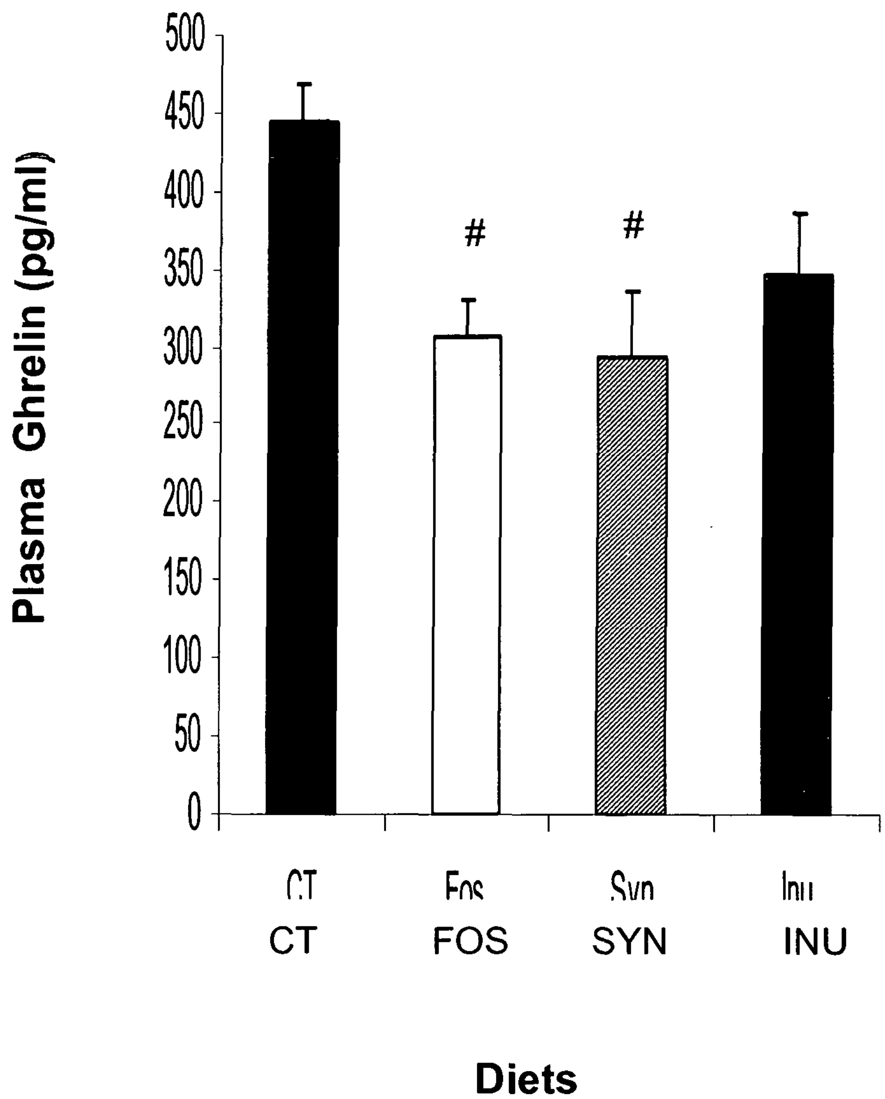


Mean value +/- ESM, n = 6. (#) significant *versus* CT ($p$<0.05)

Diets: CT : Control group (Feed UAR-AO4)

FOS : UAR-AO4 + 10 % FOS (RAFTILOSE® P95)

SYN : UAR-AO4 + 10 % RAFTILOSE® SYNERGY1

INU : UAR-AO4 + 10 % Inulin (RAFTILINE® HP)

European Patent Office

# EUROPEAN SEARCH REPORT

Application Number
EP 03 02 2007

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | NEDVÍDKOVÁ, JARA: "Loss of Meal-Induced decrease in Plasma Ghrelin Levels in Patients with Anorexia Nervosa" THE JOURNAL OF CLINICAL ENDOCRINOLOGY & METABOLISM, vol. 88, no. 4, 2003, pages 1678-1682, XP008027633 * page 1678, column 1, paragraph 2 * * page 1679, column 2, paragraph 4 * * page 1680, column 1, paragraph 2 * --- | 1-13 | A23L1/308 A61P3/04 |
| A | WREN A M ET AL: "Ghrelin enhances appetite and increases food intake in humans" JOURNAL OF CLINICAL ENDOCRINOLOGY AND METABOLISM, NEW YORK, NY, US, vol. 86, no. 12, December 2001 (2001-12), pages 5992-5995, XP002265244 ISSN: 0021-972X * page 5992, column 1, paragraph 1 - paragraph 3 * * page 5994, column 2, paragraph 2 * * page 5995, column 1, paragraph 4 - paragraph 5 * ----- | 1-13 | |
| | | | TECHNICAL FIELDS SEARCHED (Int.Cl.7) |
| | | | A23L |

~~The present search report has been drawn up for all claims~~

| Place of search | Date of completion of the search | Examiner |
|---|---|---|
| THE HAGUE | 18 March 2004 | Inceisa, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

3

EPO FORM 1503 03.82 (P04C01)

European Patent Office

Application Number

EP 03 02 2007

## CLAIMS INCURRING FEES

The present European patent application comprised at the time of filing more than ten claims.

- [X] Only part of the claims have been paid within the prescribed time limit. The present European search report has been drawn up for the first ten claims and for those claims for which claims fees have been paid, namely claim(s):

  11-13

- [ ] No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

- [ ] All further search fees have been paid within the fixed time limit. The present European search report has been drawn up for all claims.
- [ ] As all searchable claims could be searched without effort justifying an additional fee, the Search Division did not invite payment of any additional fee.
- [ ] Only part of the further search fees have been paid within the fixed time limit. The present European search report has been drawn up for those parts of the European patent application which relate to the inventions in respect of which search fees have been paid, namely claims:
- [ ] None of the further search fees have been paid within the fixed time limit. The present European search report has been drawn up for those parts of the European patent application which relate to the invention first mentioned in the claims, namely claims: